(19) Europäisches Patentamt
European Patent Office
Office européen des brevets

(11) Numéro de publication: **0 150 638**
**A1**

(12) **DEMANDE DE BREVET EUROPEEN**

(21) Numéro de dépôt: **84402398.6**

(22) Date de dépôt: **23.11.84**

(51) Int. Cl.⁴: **A 61 K 47/00**, A 01 N 53/00

(30) Priorité: **25.11.83 FR 8318881**

(43) Date de publication de la demande: **07.08.85 Bulletin 85/32**

(84) Etats contractants désignés: **AT BE CH DE FR GB IT LI LU NL SE**

(71) Demandeur: **CENTRE DE RECHERCHE D'APPLICATIONS PHARMACEUTIQUES LABORATOIRES APPLIPHARM, 77, avenue des Champs-Elysées, F-75008 Paris (FR)**

(72) Inventeur: **Gerbe, Marcel, 74 Avenue des Champs-Elysées, F-75008 Paris (FR)**

(74) Mandataire: **Phélip, Bruno et al, c/o Cabinet Harlé & Phélip 21, rue de la Rochefoucauld, F-75009 Paris (FR)**

(54) **Composition à base de pyréthroides pour le traitement local des parasitoses cutanées.**

(57) Nouvelle composition pharmaceutique pour application locale contenant:

a) 0,1 à 10% d'un pyréthroïde de synthèse,

b) 0,5 à 50% d'un agent synergique des pyréthroïdes insecticides,

c) 20 à 80% d'un monoéther de diéthylèneglycol,

d) 20 à 80% de glycérides d'acides gras polyéthoxylés.

Application au traitement de la gale et des aoûtats.

EP 0 150 638 A1

0150638

**Composition à base de pyréthroïdes pour le traitement local des parasitoses cutanées.**

La présente invention concerne une nouvelle composition pharmaceutique pour le traitement topique des parasitoses cutanées et notamment de la gale et de la trombidiose automnale (aoûtats).

Les produits utilisés pour traiter la gale, affection de la peau due à un parasite du type sarcopte, sont en général appliqués par badigeonnage mais après un traitement mécanique superficiel de façon à permettre aux produits actifs de pénétrer dans les galeries creusées dans la couche cornée par les parasites et dans lesquelles ils se logent. Ceci impose de baigner le malade avant l'application avec un pinceau de produits à base de disulfirame, de lindane, de DDT et autres. En outre, ces produits sont très irritants, et leur emploi est douloureux pour le patient, qui doit les supporter un certain temps car leur action n'est pas rapide. Enfin, ces produits sont toxiques et une toxicité systémique se manifeste fréquemment notamment sur les structures nerveuses du malade.

On a maintenant trouvé une composition qui ne présente pas de tels inconvénients. L'agent parasiticide est particulièrement efficace et agit rapidement, le véhicule auquel il est associé est tel que le principe actif pénètre dans l'épiderme où se trouvent les galeries creusées par l'insecte et où logent la femelle et les oeufs, sans qu'il soit nécessaire de les mettre à nu par un prétraitement mécanique, sans pour autant pénétrer dans le derme, puisqu'on n'a retrouvé qu'une très faible quantité du principe actif dans le sang des

patients après un traitement par une telle composition.

Selon l'invention, la composition pharmaceutique pour le traitement local des parasitoses cutanées comprend :

a) de 0,1 à 10 % en poids d'isomère insecticide d'un pyréthroïde, ester de l'acide gem-diméthyl-cyclopropane carboxylique de formule I :

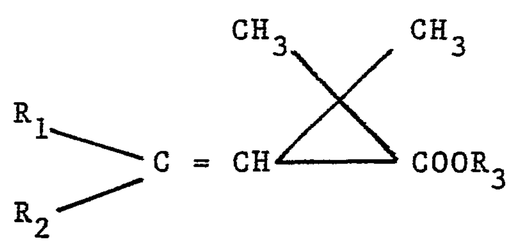

dans laquelle :

$R_1$ et $R_2$ représentent un groupe méthyle ou un halogène ou

$R_1$ est le groupe méthyle, alors que $R_2$ est le groupe carboxyméthyle,

et $R_3$ est 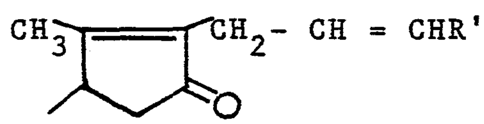

formule dans laquelle

R' représente H, $CH_3$, $C_2H_5$ ou $CH = CH_2$

ou $R_3$ est 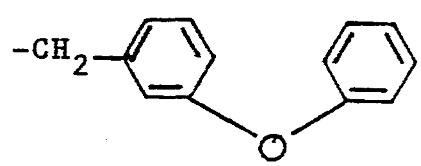

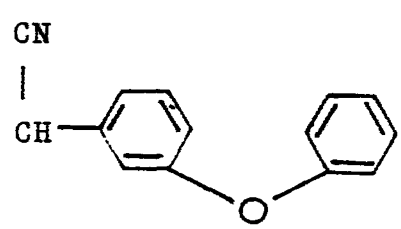

ou 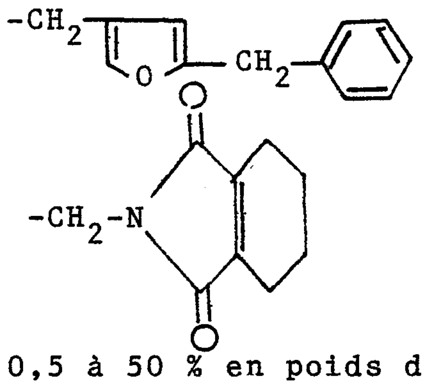

b) de 0,5 à 50 % en poids d'un synergiste des pyréthroïdes insecticides, à groupe méthylène dioxyphényle de formule :

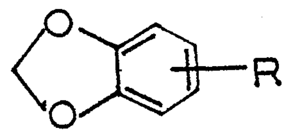

dans laquelle :

R est une chaîne alkyle oxygénée ou soufrée;

c) de 20 à 80 % en poids de monoalkyléther de diéthylène glycol répondant à la formule $C_nH_{2n}$ $-OCH_2CH_2OCH_2CH_2OH$, dans laquelle n est 1 à 4 ;

d) de 20 à 80 % en poids de "glycérides d'acides gras polyéthoxylés".

Selon une forme particulière de l'invention, on ajoute aux composants précédents de 1 à 20 % en poids d'un alcool inférieur, tel que l'alcool éthylique ou isopropylique.

Les compositions pharmaceutiques selon l'invention sont particulièrement adaptées au traitement local de la gale et seront présentées notamment sous forme d'aérosol, associées aux gaz propulseurs habituels et conditionnées dans des récipients courants,ou encore sous forme de lotion (après dilution).

Les pyréthroïdes insecticides sont des composés

de synthèse bien connus de l'homme du métier et différentes revues des études effectuées des relations entre la structure chimique et stéréochimique et l'activité insecticide de ces composés ont été publiées. On peut citer notamment "Structure-activity relationships of some pyrethroids in rats" de R.D. Verschoyle et W.N. Aldridge, publié dans Archives of Toxicology, 45, p. 325-329 (1980) ou "Quantitative Structure-activity relationships of pyrethroid insecticids" de Martin G. Ford, publié dans Pestic. Sci. 10 p. 39-49 (1979).

Les pyréthroïdes de synthèse particulièrement préférés selon l'invention sont ceux présentant un bon index thérapeutique, c'est-à-dire une forte activité insecticide, (que l'on peut mettre par exemple en évidence in vitro sur des acariens proches du sarcopte de la gale, ce dernier ne pouvant être cultivé in vitro, tels que celui de la tique du chien ou ceux des poussières domestiques), avec une toxicité minimale, (étudiée de la manière habituelle chez les animaux comme rats, souris, chiens).

Selon l'invention, afin de diminuer les risques inhérents à la toxicité des pyréthrinoïdes pour les animaux supérieurs et l'homme, on l'utilise associé à un agent synergiste connu, qui en augmente l'activité beaucoup plus que la toxicité. Certains de ces composés sont décrits dans l'ouvrage John E. CASIDA "Pyrethrum" Academic press (1973) ou dans "Mode of action of Synergists in enhancing the insecticidal activity of pyrethrum and pyrethroïds" de Izuru Yamamoto, p. 195-210. Les composés particulièrement préférés sont ceux

comportant un groupe méthylène dioxyphényle mais d'autres peuvent aussi convenir. On peut citer les composés suivants, comme exemple (dénomination commune du Merck Index) : piperonyl butoxyde et analogues, Sesamin et analogues, sulfoxyde, synepirin 500 et analogues, octachlorodipropyléther, et dérivés phosphorés.

Il était important de combiner un tel principe actif à un véhicule dans lequel il soit soluble, stable, mais aussi un véhicule qui assure la pénétration du principe actif dans l'épiderme où se logent le parasite et ses oeufs, dans des galeries creusées dans la couche cornée, et qui limite simultanément la pénétration du même principe actif dans le derme d'où il pourrait passer dans la circulation sanguine, ce qui pourrait être la cause de manifestations d'effets secondaires chez le patient.

La composition résultante doit en outre s'étaler facilement sur la peau, donc être suffisamment fluide, et surtout ne pas être irritante et donc douloureuse pour les malades. On a trouvé qu'une association d'un monoéther du diéthylène glycol, favorisant la pénétration percutanée des produits auxquels il est mélangé, et de glycérides d'acides gras polyéthoxylés (huiles végétales naturelles polyéthoxylées) avait ces propriétés. Le véhicule préféré est un mélange de monoéther éthylique de diéthylèneglycol et de "glycérides oléiques polyoxyéthylénés", vendus par les Etablissements GATTEFOSSE. Saint-Priest-France sous le nom LABRAFIL(R) M 1944 CS (marque déposée). Enfin, un alcool, aqueux ou non, peut être introduit dans

la composition, notamment dans le cas où elle est destinée à une utilisation en lotion ; il en aumente la fluidité. On préfère l'éthanol aqueux à 95 % dont l'action antiseptique est connue et complète avantageusement les propriétés thérapeutiques de la composition.

La composition selon l'invention est utilisée de préférence sous forme aérosol, par pulvérisation soit à partir d'un vaporisateur actionné manuellement, soit à partir d'un récipient, avec une valve, contenant un gaz liquéfié, qui sert de propulseur, selon une technique connue de l'homme de l'art.

La quantité de la composition pharmaceutique appliquée sur chaque patient par pulvérisation ou badigeonnage dépend de l'étendue des lésions. Pour le traitement de la gale, on appliquera par pulvérisation généralement sur l'ensemble du corps, en évitant le visage, 80 g de la composition pour un adulte et 40 g pour un enfant de moins de 13 ans, et la laissera agir sur la peau durant 12 heures environ, avant lavage.

Dans ce qui suit, on décrit deux exemples de composition pharmaceutique selon l'invention et leur utilisation dans le traitement de la gale humaine, mais l'invention ne saurait être limitée à ces modes de réalisation et type d'application thérapeutique.

EXEMPLE 1

On introduit à température ambiante, dans un récipient, 9,2 g d'éthanol aqueux à 95 % (v/v), 18,4 g de Labrafil(R) M 1944 CS, puis 7,2 g de O-méthylènedioxy-3,4-propyl-6 benzyl O-butyldiéthylèneglycol (ou butoxyde de pipéronyle) et

0,90 g de d-transchrysanthémate de d-alléthrolone et enfin 64,3 g de monoéther éthylique du diéthylène glycol. Le mélange est homogénéisé par agitation modérée quelques minutes puis conditionné de façon classique dans des unités de pulvérisation, de type boîtier aérosol, à raison de 56 g de mélange et 24 g de gaz butane liquéfié par boîtier de 300 ml , comme ceux commercialisés par PAMASOL.

EXEMPLE 2

On dissout à température ambiante, dans un mélange de 10 ml d'éthanol aqueux à 95 % (v/v) 20 ml de Labrafil(R) M 1944 CS et 70 ml d'éther monoéthylique du diéthylèneglycol, 0,8 g de d-transchrysanthémate de d-alléthrolone et 6,5 g de butoxyde de pipéronyle.

Après quelques minutes d'agitation pour homogénéiser, la lotion est prête pour être conditionnée en unités de 60 ml environ, dose convenant à un malade adulte.

La toxicité de chacun des constituants des compositions de l'invention est bien connue, et on a constaté qu'il n'y avait pas de potentialisation de celle-ci, par exemple chez le rat, après administration intraveineuse et que chez le lapin après administration répétée par voie dermique, très peu du principe actif se retrouve dans le plasma de l'animal.

Le pouvoir irritant des compositions selon l'invention a été étudié chez le lapin, par un test d'irritation cutanée primaire, dont les conditions de réalisation sont définies dans le Journal Officiel de la République Française du 21 avril 1971, complété par celui du 5 juin 1973. Il a été

comparé à celui des compositions où des pyréthrines naturelles remplaçaient les pyréthrinoïdes de l'invention et à celui de compositions résultant du mélange des pyréthrinoïdes et d'huile de vaseline ou d'isododécane.

Les résultats obtenus figurent dans le tableau I ci-après.

On constate que le pyréthrinoïde de l'exemple 1 est beaucoup moins irritant que d'autres isomères de l'alléthrine et qu'une pyréthrine naturelle (tableau I) et que d'autre part, les véhicules selon l'invention donnent des compositions nettement moins irritantes que celles contenant un hydrocarbure, couramment utilisé jusqu'à présent.

Dans ces essais, l'agent synergiste n'aviat pas été introduit, mais on a pu constater ultérieurement, qu'il ne modifiait pas le pouvoir irritant de telles compositions.

TABLEAU I

| Constituants | Pourcentage en poids dans la formule | | | | | | | | | | | | | | | |
|---|---|---|---|---|---|---|---|---|---|---|---|---|---|---|---|---|
| | I | II | III | IV | V | VII | VIII | IX | X | XI | XII | XIII | XIV | XV | XVI | XVII |
| d-transchrysanthémate de d-alléthrolone | - | 4,8 | - | - | - | - | - | 4,8 | 4,8 | 4,8 | 4,8 | | - | - | - | - |
| d-transchrysanthémate de dl-alléthrolone | 9 | - | - | 9 | 9 | 9 | 9 | - | - | - | - | - | - | - | - | - |
| Pyréthrine naturelle | - | - | 11 | - | - | - | - | - | - | - | - | 11 | 11 | 11 | 11 | 11 |
| Ethanol | - | - | - | - | 9,1 | 9,1 | - | 9,52 | 9,52 | 9,52 | - | 8,9 | 8,9 | 8,9 | - | - |
| Labrafil M 1944 CS | - | - | - | - | 9,1 | 18,2 | 9,1 | 9,52 | 9,52 | 19,04 | 9,52 | 8,9 | 8,9 | 17 | 8,9 | |
| Isododécane pur | - | - | - | QSP100 | 9,1 | - | 9,1 | 9,52 | 19,04 | - | 9,52 | 8,9 | 17,8 | - | 8,9 | QSP100 |
| Ether monoéthylique du diéthylène glycol | - | - | - | - | 63,7 | 63,7 | 72,8 | 66,64 | 57,12 | 66,64 | 76,16 | 62,3 | 53,4 | 62,3 | 71,2 | - |
| Huile de vaseline | QSp100 | QSp100 | QSp100 | - | - | - | - | - | - | - | - | - | - | - | - | - |
| Indice d'irritation primaire cutanée estimé | 1,17 | 0,79 | 1,17 | 2,25 | 1,83 | 0,58 | 1,00 | 0,83 | 2,08 | 0,50 | 1,08 | 1,17 | 2,33 | 0,67 | 1,92 | 3,17 |

L'activité scabicide des compositions selon l'invention a été mise en évidence chez des malades présentant un tableau clinique caractéristique de la gale, compliquée ou non d'eczéma et de surinfection. On a pulvérisé sur plus de 50 malades adultes, en une seule fois, 80 g de la composition de l'exemple 1, en évitant le visage ; 12 heures après, les malades ont été douchés et ont changé de linge. On a vérifié 8 jours, puis 31 jours plus tard que les parasites n'étaient pas réapparus, ce qui démontre la grande efficacité des compositions de l'invention ; seuls deux cas conservaient des parasites. En outre, aucune variation des paramètres biologiques des malades qui aurait pu être imputée au traitement n'a été relevée. Les malades n'ont pas ressenti d'irritation cutanée, sauf parfois des picotements au moment de la pulvérisation du produit.

Enfin, une enquête allergologique a montré que cette composition ne présentait pas d'activité allergénique particulière.

## REVENDICATIONS

1. Composition pharmaceutique pour le traitement local des parasitoses cutanées, caractérisée en ce qu'elle contient :

a) de 0,1 à 10 % en poids d'isomère insecticide d'un pyréthroïde, ester de l'acide gem-diméthyl-cyclopropane carboxylique de formule I :

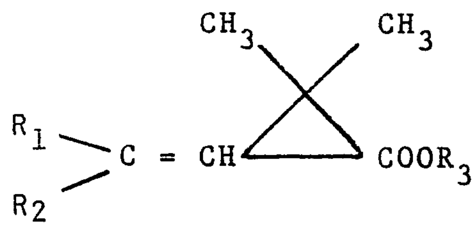

dans laquelle :

$R_1$ et $R_2$ représentent un groupe méthyle ou un halogène ou

$R_1$ est le groupe méthyle, alors que $R_2$ le groupe carboxyméthyle,

et $R_3$ est 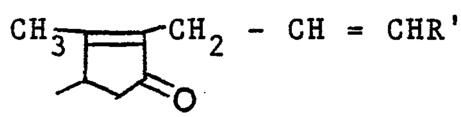

avec R' représentant H, $CH_3$, $C_2H_5$ ou $CH = CH_2$

ou $R_3$ est : 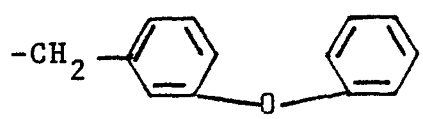

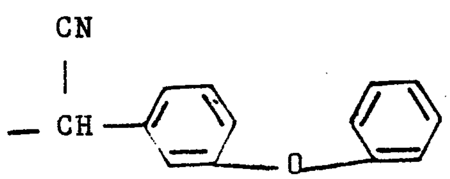

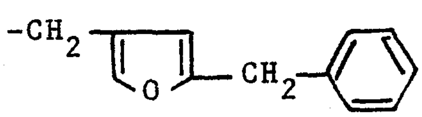

ou 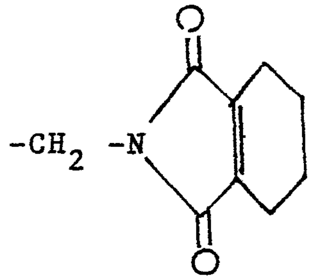

b) de 0,5 à 50 % en poids d'un synergiste des pyréthroïdes insecticides, à groupe méthylène dioxyphényle de formule :

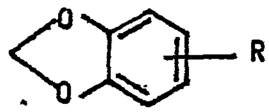

dans laquelle :
R est une chaîne alkyle oxygénée ou soufrée ;

c) de 20 à 80 % en poids de monoalkyléther de diéthylène glycol répondant à la formule $C_nH_{2n}-OCH_2CH_2OCH_2CH_2OH$, dans laquelle n est 1 à 4 ;

d) de 20 à 80 % en poids de "glycérides d'acides gras polyéthoxylés".

2. Composition pharmaceutique selon la revendication 1, caractérisée en ce qu'elle contient en outre de 1 à 20 % d'alcool éthylique ou isopropylique pur ou en mélange avec jusqu'à 30 % d'eau en volume.

3. Composition pharmaceutique selon la revendication 1 ou 2, caractérisée en ce qu'elle contient à titre de pyréthroïde le d-trans-diméthyl-2,2 (méthyl-2 propényl)-3 cyclopropanecarboxylate de d-allyl-2' hydroxy-4' méthyl-3' cyclopentène-2' one-1' de formule :

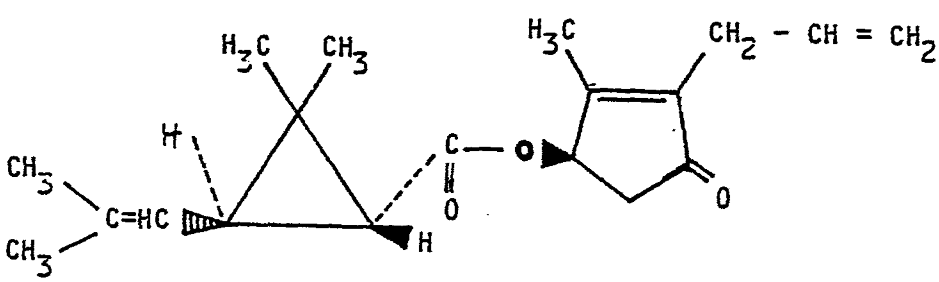

4. Composition pharmaceutique selon l'une des revendications précédentes, caractérisée en ce que l'agent synergiste du pyréthroïde est le composé de formule :

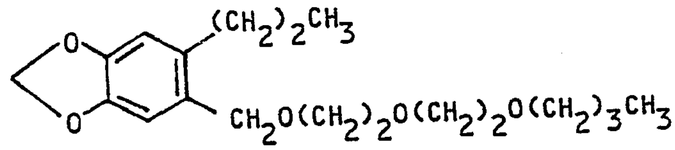

5. Composition pharmaceutique selon la revendication 2, caractérisée en ce qu'elle contient :

a) de 0,8 à 1 % en poids de d-trans diméthyl-2,2 (méthyl-2 propényl)-3 cyclopropane-carboxylate de d-allyl-2' hydroxy-4' méthyl-3' cyclopentène-2' one-1' ;

b) de 7 à 7,5 % en poids de O -méthylènedioxy-3,4 propyl-6 benzyl O-butyl diéthylène glycol ;

c) de 60 à 65 % en poids d'éther monoéthylique du diéthylène glycol ;

d) de 16 à 20 % en poids de glycérides d'acides gras polyéthoxylés ;

e) de 8 à 10 % en poids d'éthanol aqueux à 95 % en volume .

6. Composition pharmaceutique selon la revendication 5, caractérisée en ce que les glycérides d'acides gras polyéthoxylés sont du Labrafil(R) M 1944 CS.

7. Composition pharmaceutique selon l'une quelconque des revendications précédentes, caractérisée en ce qu'elle est conditionnée pour une utilisation sous forme d'aérosol, associée ou non à un gaz propulseur.

8. Composition pharmaceutique selon l'une quelconque des revendications 1 à 5, caractérisée en ce qu'elle est utilisée sous forme de lotion.

0150638

Office européen des brevets

# RAPPORT DE RECHERCHE EUROPEENNE

Numéro de la demande

EP 84 40 2398

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | GB-A- 847 517 (GATTEFOSSE)<br>* Page 1, ligne 11 - page 2, ligne 58; exemple 2 *<br>--- | 1-3,5-8 | A 61 K 47/00<br>A 01 N 53/00 |
| X | GB-A-2 095 109 (WELLCOME FOUNDATION)<br>* Page 1, lignes 21-49; exemples 1-13 *<br>--- | 1-8 | |
| Y | FR-A-2 504 779 (YOUNG)<br>* Page 2, ligne 9 - page 4, ligne 19; revendications 1-10 *<br>--- | 1-3,5-8 | |
| A | FR-A-2 484 256 (ROUSSEL-UCLAF)<br>* Page 4, ligne 26 - page 5, ligne 2; page 5, ligne 24 - page 6, ligne 8; exemple 1; revendications 1-8 *<br>--- | 1,3,7 | DOMAINES TECHNIQUES RECHERCHES (Int. Cl.4)<br>A 61 K |
| A | CHEMICAL ABSTRACTS, vol. 88, no. 23, 5 juin 1978, page 197, no. 165464j, Columbus, Ohio, US; T. YAMAGUCHI et al.: "Insecticidal aerosol formulations. I. Stability of tetramethrin and other pyrethroids in water-based aerosol formulations" & BOCHU KAGAKU 1977, 42(3), 104-110<br>* Abrégé *<br>----- | 1,3 | |

Le présent rapport de recherche a été établi pour toutes les revendications

| Lieu de la recherche | Date d'achèvement de la recherche | Examinateur |
|---|---|---|
| LA HAYE | 13-03-1985 | CONTET F.L.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82